# EUROPEAN PATENT SPECIFICATION

(11) **EP 1 029 927 B1**
(45) Date of publication and mention of the grant of the patent: **23.10.2002**
(21) Application number: 99103099.0
(22) Date of filing: 17.02.1999
(51) Int. Cl.: C12P 19/38, C12R 1/645, C07H 19/052, A61K 31/70

(54) **Process for producing mizoribine by Eupenicillium javanicum**
Verfahren zum Herstellen von Mizoribin durch Eupenicillium javanicum
Procédé de préparation de mizoribine par Eupenicillium javanicum

(43) Date of publication of application: 23.08.2000
(73) Proprietor: GNOSIS SRL, 21050 Cairate (Varese) (IT)
(72) Inventor: Benedetti, Alberto, 20063 Cernusco sul Naviglio (MI) (IT); Colombo, Elisabetta, 21011 Casorate Sempione (VA) (IT); Nichele, Marina, 20020 Vanzaghello (MI) (IT); Pagani, Hermes, 20099 Sesto San Giovanni (MI) (IT)
(74) Representative: Mancini, Vincenzo, Dr.

(56) References cited:
- GB-A- 1 363 622
- MIZUNO K. ET AL.: "STUDIES ON BREDININ. I ISOLATION, CHARACTERIZATION AND BIOLOGICAL PROPERTIES" THEJOURNAL OF ANTIBIOTICS, vol. XXVII, no. 10, 1974, pages 775-782, XP002107668
- PATENT ABSTRACTS OF JAPAN vol. 097, no. 011, 28 November 1997 (1997-11-28) & JP 09 194497 A (BANYU PHARMACEUT CO LTD), 29 July 1997 (1997-07-29)

## Description

The present invention relates to a process for producing mizoribine. Particularly, the invention concerns a fermentation process for producing mizoribine which comprises cultivating a microorganism belonging to genus Eupenicillium.

Mizoribine, also known as bredinin (5-hydroxy-1-β-D-ribofuranosyl-1H-imidazole-4-carboxamide), is a known nucleoside antibiotic, endowed with cytotoxic and immunosuppressive activity, currently produced by Eupenicillium brefeldianum (US 3,888,843), which was reported to provide a broth showing a potency of 350 µg/ml (Mizuno et al., Journal of Antibiotics, vol. XXVII, No. 10, pp. 775-782, 1974).

It has now been found that mizoribine can be prepared by means of a microbiological process which envisages fermentation of Eupenicillium javanicum, identified in our strain collection as GN-83, and filed on September 24, 1998 with the "Deutsche Sammlung von Mikroorganismen" (DSMZ), accession number: DSM 12615.

The producing organism was isolated from a soil sample collected at Aurangabad (India) and classified according to the criteria proposed by J.I. Pit in "The Genus Penicillium", Academic Press, London 1979.

The characteristics of the microorganism, colonies and morphology, are herebelow illustrated. The course of growth on a malt extract agar (MEA) was evaluated after 7 days of incubation at 25°C.

The colonies have an average diameter equal to 43 mm with a uniform, woolly, velvet-like, white-edged surface, showing a crown equal to 4-5 mm, and uniformly yellow in the centre. Frequently, the appearing of sectors with white mycelium, and the absence of exudates, present in the white sectors, was observed.

The back of the colonies is cream-yellow coloured with sectors showing a darker pigmentation. Diffusible pigments are absent.

The cleistothecia, differentiated only on the cultural substrate MEA, come to maturation after 15 days of incubation. They show a spherical shape and have dimensions ranging between 100 and 200 µm; they are cream-yellow coloured and form a uniform sub-mycelial layer.

The ascospores, contained in ascocarps, have a curved shape and dimensions ranging between 3.0 and 3.5 µm. They do neither show any evident surface ornamentation nor any sulci.

In Eupenicillium javanicum, the differentiation of the amorphous reproductive structures, referable to genus Penicillium, starts, after 15 days of culturing, only on the MEA substrate.

The conidiophores, numerically poor, form themselves on aerial hyphae differentiating a stipe having a variable length, however longer than 30 µm.

The penicillia are uniseriated and have a low number (3-6) of bottle-like conidiospores per conidiophorous head. The chains of the conidia at the top of the conidiospores have a variable length.

As the colony ages, both the number of the conidiophores and the length of the chains of the conidia tend to increase in some sectors of the colony. The presence of the conidiophores on MEA, when the colony is aged one month, causes a light greenish shade taken by some areas of the colony. The conidia are spherical, smooth-walled and have dimensions ranging between 1.5 and 2.5 µm.

The invention provides a fermentation process for producing mizoribine which comprises fermentation in the presence of a strain of Eupenicillium javanicum, carried out, in aerobic conditions, in a nutrient liquid medium containing an assimilable source of carbon and nitrogen. The process of the invention can be advantageously carried out at a temperature between 23°C and 33°C, for a period of time varying between 3-7 days. Further, carrying out the fermentation in the presence of added mineral powder composition, can be of some advantage.

Still further, the invention encompasses the purification of mizoribine, and/or the preparation of a pharmaceutically acceptable composition thereof, as it results from the fermentation broth.

Fermentation is carried out in the liquid phase, under stirring, at a temperature ranging between 23°C and 33°C, preferably 25°C-29°C, for 3-7 days, preferably 4-6 days.

The strain is preferably pre-cultivated and then inoculated in aerated fermenters in order to get remarkable amounts of mizoribine.

The culture medium used for the growth of Eupenicillium javanicum consists of sources of carbon, nitrogen and, preferably, of mineral salts. The sources of carbon can be, for instance, starch, dextrose, dextrin, glucose, fructose, glycerine, saccharose, mannitol, mannose, and the like; the nitrogen sources can be, for instance, malt extract, corn steep liquor, enzymatic hydrolysate of casein, soya flour, dry yeast, peptone, soy peptone, meat extract, nitrate, amino acids, casein, and the like. Good results are also obtained using ammonium salts such as ammonium nitrate and ammonium sulphate. The mineral salts used for the production can vary depending on the culture medium; the soluble inorganic salts which provide sodium, potassium, magnesium, sulphate, chloride, nitrate ions, can be used. Such salts can be, for instance, monobasic potassium phosphate, magnesium sulphate, monobasic potassium sulphate, sodium nitrate. The addition of calcium carbonate may also be useful.

Fermentation can be carried out in a flask and in fermenters of different capacity.

### Analytical methods

### CHROMATOGRAPHY (HPLC)

Beckman chromatographic equipment system:
- 126 pump;- 168 detector;- 507e autosampler
The volume of the injection loop was 50 µl. A stainless-steel column (15 cm x 6.0 mm I.D.) packed with 5 µm LICHROSORB was used at room temperature. The mobile phase was 70 mM monobasic potassium phosphate (pH 2.5)-acetonitrile (30:70), delivered at a flow-rate of 1.0 ml/min. The column effluent was monitored at 280 nm with an absorbance sensitivity of 0.050.

### SAMPLE PREPARATION

After contrifugation (10 min. at 3.000 rpm), a 50 µl opportunately diluted aliquot of the clear supernatant was injected into the HPLC system.

### QUANTITATION

A stock solution of mizoribine was prepared in water at a concentration of 100 µg/ml. Using the mizoribine standard solutions, samples of broth culture were tested with concentrations ranging from 25 to 100 µg/ml.

After concentration of the organic phase, optionally dried and re-suspended in the same solvent or a mixture of solvents, (for instance, butanol / acetic acid / water), a crude precipitate is obtained containing mizoribine.

The purification of the crude product, following fermentation, can take place using, for instance, chromatographic columns using, as eluent, mixtures of water and organic solvents such as acetone, acetic acid, methanol, butanol, chloroform.

### EXAMPLE 1

### Strain growth

The strain of Eupenicillium javanicum was stored on an agar-slant having the following composition (MEA: Malt Extract Agar):

| | |
|---|---|
| Malt extract (Difco) | 20.0 g/l |
| Peptone (Difco) | 1.0 g/l |
| Dextrose (Roquette) | 20.0 g/l |
| Agar (Difco) | 20.0 g/l |
| Distilled water | 1000 ml |

The slants were incubated at 26°C for 15-18 days.

About one third of the growth was taken off by scraping the surface with 5 ml of distilled water to get a spore suspension.

### Pre-culture

The suspension resulting from the strain growth above described was inoculated in a shake-flask as a pre-culture.

The culture in the shake-flask had the following composition:

| | |
|---|---|
| Enzymatic hydrolysate of casein (Difco) | 3 g/l |
| Corn steep liquor (Roquette) | 1 g/l |
| Dextrose (Roquette) | 20 g/l |
| Distilled water | 1000 ml |

Sterilization was carried out at 121°C for 20 minutes.
The medium was distributed in 500 ml flasks, each having two baffles and containing 100 ml of the above medium. The flasks were incubated at 26°C for 24 hours on a rotatory shaker at 250 rpm.

A second pre-culture was carried out by inoculating an amount equal to 2% of the first pre-culture in a 2 1 flask, with 3 baffles, containing 750 ml of the above medium.

The flask was incubated at 26°C for about 48 hours on a rotatory shaker at 200 rpm.

### Fermentation

About 2% of the second pre-culture was used to inoculate a jar fermenter containing 15 l of nutrient medium having the following composition:

| | |
|---|---|
| Ammonium sulphate (Merck) | 10 g/l |
| Ammonium nitrate (Merck) | 10 g/l |
| Monobasic potassium phosphate (Merck) | 2 g/l |
| Magnesium sulphate heptahydrate (Merck) | 0.2 g/l |
| Corn steep liquor (Roquette) | 20 g/l |
| Dextrose (Roquette) | 50 g/l |
| Tap water | 1000 ml |

The fermentations were incubated in aerobic conditions, under stirring, at 600 rpm with an aeration of 1 v/v/min at 26°C-28°C, for 96 h.

The production of mizoribine was tested, at intervals, on the culture-broth filtrate by HPLC.

The highest potency, equal to 600-700 µg/ml, was registered after 4-5 days.

### EXAMPLE 2

### Strain growth

The strain of Eupenicillium javanicum was stored on an agar slant having the following composition:

| | |
|---|---|
| Malt extract (Difco) | 20.0 g/l |
| Monobasic potassium phosphate | 1.0 g/l |
| Dextrose (Roquette) | 20.0 g/l |
| Magnesium sulphate heptahydrate (Merck) | 0.2 g/l |
| Corn steep liquor (Roquette) | 20.0 g/l |
| Agar (Difco) | 20.0 g/l |
| Distilled water | 1000 ml |

The slants were incubated at 26°C for 15-20 days.

About one third of the growth was taken off by scraping the surface with 5 ml of distilled water to get a spore suspension.

### Pre-culture

The spore suspension resulting from the strain growth was inoculated in a shake-flask containing a nutrient medium having the following composition:

| | |
|---|---|
| Enzymatic hydrolysate of casein | 2 g/l |
| Soy peptone | 5 g/l |
| Dextrose | 10 g/l |
| Distilled water | 1000 ml |

Sterilization was carried out at 121°C for 20 minutes.
The medium was distributed in 500 ml flasks, each having two baffles and containing 100 ml of the above medium.

The flasks were incubated at 26°C for about 40 hours on a rotatory shaker at 250 rpm.

A second pre-culture was prepared and incubated as in Example 1.

### Fermentation

After the growth, the pre-culture was inoculated in a 15 l fermenter containing a nutrient medium having the following composition:

| | |
|---|---|
| Ammonium sulphate | 5 g/l |
| Sodium nitrate | 10 g/l |
| Monobasic potassium phosphate | 2 g/l |
| Magnesium sulphate heptahydrate | 0.2 g/l |
| Soy peptone | 20 g/l |
| Saccharose | 60 g/l |
| Tap water | 1000 ml |

The fermentations were incubated in the same conditions as in Example 1 and the production of mizoribine, tested by HPLC, showed a potency of 750-800 µg/ml after 4 days.

### EXAMPLE 3

### Extraction

50 l of culture-broth (resulting from Example 1) having a pH of 5.6 and containing 700 µg/ml of mizoribine were filtered on Wathman® paper to obtain 40 l of a limpid filtrate.
The filtrate was passed through a column (⌀ 12 cm) using 8 l of XAD 1180 (ROHM & HAAS) resin with a flow of 330 ml/min in order to decolorise the broth: the decolorisation was followed by reading the absorbance at 420 nm. The resin was then washed with 20 l of water with the same flow as above.
60 l of the resulting decolorised liquid (pH=5.8) were adjusted to pH = 9.0 by adding 20% NaOH.
The liquid was passed through a 30 l Relite 2AS (RESINDION S.R.L.) (OH form) column (Ø 20 cm) with a flow of 500 ml/min in order to absorb mizoribine.

The resin was washed with 120 l of demineralized water, then eluted with 2% acetic acid collecting 10 l fractions.

The active fractions, tested with HPLC, were collected and concentrated under vacuum in order to obtain 220 g of an oily residue.

This residue was mixed with 400 ml of methanol and 1 litre of acetone and precipitated, letting it stand 5 hours at 4°C. The precipitate was washed with acetone and dried under vacuum to obtain 150 g of crude mizoribine (purity: 15%). The powder obtained was suspended in a solvent mixture butanol:acetic acid:water (10:1:2) and purified in a Silicagel 60 column (Ø 7 cm) packed with the same solvent mixture.

500 ml fractions were collected, the richest ones being concentrated under vacuum till 33 g of an oily residue (purity: 44%) was obtained and then suspended again with 30 ml of methanol and precipitated with 90 ml of acetone.

The precipitate was washed under vacuum with acetone to obtain 28 g of a grey powder which was suspended in methanol and charged through a 1 litre Silicagel 60 column (⌀ 4 cm) packed with a solvent mixture of chloroform:methanol:acetic acid (30:1:1), eluting in gradient till a mixture of 1:7:7 was obtained. 500 ml fractions were collected, the richest ones being concentrated under vacuum to obtain 14.5 g of an oily residue (purity: 85%) to which an equal volume of acetone was added letting it stand overnight at 5°C.
Colourless mizoribine crystals were obtained (11 g).

### EXAMPLE 4

### Extraction.

50 l of culture-broth (as it results from Example 1) (pH = 5.6) containing 700 µg/ml of mizoribine were filtered under vacuum on Dicalite (DICALITE EUROPE SUD).

40 l of limpid filtrate were adjusted to pH 4.5 by adding 20% H₂SO₄ and then passed through a 5 1 CM SEPHAROSE FAST FLOW column (⌀ 12 cm) packed with 20 l of 0.05M acetate buffer at pH 4.5.
The filtrate was absorbed at a flow of 83 ml/min then washed with 5 l of 0.05M acetate buffer (pH 4.5) and eluted in gradient, mixing 25 l of 0.05 acetate buffer (pH 4.5) with further 25 l of 0.05 acetate buffer (pH 4.5) and 0.5M NaCl.

1 litre fractions were collected, the richest ones being concentrated under vacuum to obtain 45 g of a grey powder (purity: 55%).

The powder suspended in methanol was purified on a 1 litre Silicagel 60 column (⌀ 4 cm) packed with a solvent mixture of chloroform/methanol/acetic acid (1:7:7).

500 ml fractions were collected and the richest ones were concentrated under vacuum till 18 g of colourless crystals of mizoribine were obtained (purity: 97%).

### EXAMPLE 5

### Extraction

50 l of culture-broth (as it results from Example 1) (pH = 5.6) containing 700 mcg/ml of mizoribine were filtered under vacuum on Dicalite (DICALITE EUROPE SUD).

40 l of limpid filtrate were purified also with ultrafiltration through a membrane of sulphonic polyether. The liquid was adjusted to pH = 9.0 by adding 20% NaOH.

The liquid was passed through a 30 l Relite (RESINDION S.R.L.) 500 ml/min. in order to absorb the mizoribine.

The resin was washed with 120 l of demineralized water, then eluted with 2% acetic acid, collecting 10 l fractions.

The active fractions, tested with HPLC, were collected and concentrated under vacuum in order to obtain 150 g of oil.

This residue was mixed with 150 ml of methanol and 300 ml of acetone and precipitated, letting it stand 5 hours at 4°C. The precipitate was washed with acetone and dried under vacuum to obtain 110 g of crude mizoribine (purity 20%). The powder obtained was suspended in a solvent mixture butanol:acetic acid:water (10:1:2) and purified in a Silicagel 60 column (⌀ 7 cm) packed with the same solvent mixture.

500 ml fractions were collected, the richest ones were concentrated under vacuum till a 30 g oily residue (purity: 55%) was obtained and then suspended again in 10 ml of methanol and finally precipitated with 30 ml of acetone.

The precipitate was washed under vacuum with acetone and let stand overnight under vacuum.

Colourless mizoribine crystals were obtained (13 g).

## Claims

1. A fermentation process for producing mizoribine which comprises fermentation in the presence of a strain of Eupenicillium javanicum, carried out, in aerobic conditions, in a nutrient liquid medium containing an assimilable source of carbon and nitrogen.

2. A process according to claim 1, wherein the strain is pre-cultured.

3. A process according to claim 1 or 2, carried out at a temperature between 23°C and 33°C, for a period of time varying between 3-7 days.

4. A process according to claim 3, carried out at a temperature between 25°C and 29°C, for a period of time varying between 4-6 days.

5. A process according to any of the foregoing claims, wherein the fermentation is carried out in the presence of added mineral salts.

6. A process according to any of the foregoing claims, which comprises the purification of mizoribine and/or the preparation of a pharmaceutically acceptable composition thereof.

## Patentansprüche

1. Fermentationsverfahren zur Herstellung von Mizoribin durch Fermentation in Anwesenheit eines Stamms von Eupenicillium javanicum, durchgeführt unter aeroben Bedingungen, in einem flüssigen Nährmedium, das eine assimilierbare Kohlenstoff- und Stickstoffquelle enthält.

2. Verfahren nach Anspruch 1, wobei der Stamm vorkultiviert wurde.

3. Verfahren nach Anspruch 1 oder 2, durchgeführt bei einer Temperatur zwischen 23°C und 33°C während einer Zeitdauer, die zwischen 3 und 7 Tagen variiert.

4. Verfahren nach Anspruch 3, durchgeführt bei einer Temperatur zwischen 25°C und 29°C, während einer Zeitdauer, die zwischen 4 und 6 Tagen variiert.

5. Verfahren nach einem der vorhergehenden Ansprüche, wobei die Fermentation in Anwesenheit zugesetzter Mineralsalze durchgeführt wird.

6. Verfahren nach einem der vorhergehenden Ansprüche, das die Reinigung des Mizoribins und/oder die Herstellung einer pharmazeutisch annehmbaren Zubereitung davon umfasst.

## Revendications

1. Procédé de fermentation pour la production de mizoribine qui comprend la fermentation en présence d'une contrainte d'Eupenicillium de javanicum, effectué, en conditions aérobies, dans un milieu liquide de substances nutritives contenant une source assimilable de carbone et d'azote.

2. Procédé selon la revendication 1, dans lequel la contrainte est précultivée.

3. Procédé selon la revendication 1 ou 2, effectué à une température entre 23° C et 33° C, pendant une période variant de 3 à 7 jours.

4. Procédé selon la revendication 3, effectué à une température entre 25° C et 29° C, pendant une période variant de 4 à 6 jours.

5. Procédé selon l'une quelconque des revendications précédentes, dans lequel la fermentation est effectuée en présence des sels minéraux supplémentaires.

6. Procédé selon l'une quelconque des revendications précédentes qui comprend la purification de mizoribine et /ou en une préparation d'une composition pharmaceutiquement acceptable de celle-ci.
